(19) Europäisches Patentamt
European Patent Office
Office européen des brevets

(11) **EP 2 952 498 A1**

(12) **EUROPEAN PATENT APPLICATION**

(43) Date of publication:
09.12.2015   Bulletin 2015/50

(51) Int Cl.:
*C07C 1/24* $^{(2006.01)}$   *B01J 27/18* $^{(2006.01)}$

(21) Application number: 14180473.2

(22) Date of filing: 11.08.2014

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.06.2014   SG 201402964**

(71) Applicant: **LANXESS Deutschland GmbH 50569 Köln (DE)**

(72) Inventors:
- **Gottlieb, Nadine 50859 Köln (DE)**
- **Dreisbach, Claus 42799 Leichlingen (DE)**
- **Schlenk, Stefan 86916 Kaufering (DE)**

(54) **Dehydration of 2,3-butanediole**

(57)   The present invention is directed to the dehydration of 2,3-butanediole to butadiene using as catalysts mixed alkaline earth metal phosphates or pyrophosphates or lithium alkaline earth phosphates or pyrophosphates.

EP 2 952 498 A1

## Description

The present invention is directed to the dehydration of 2,3-butanediol to butadiene (BDE) using as catalysts mixed alkaline earth metal phosphates or pyrophosphates or lithium alkaline earth phosphates or pyrophosphates.

## Background of the invention

Butadiene is mainly used in the manufacture of synthetic resins, plastics and other valuable products. The market demand is estimated to grow at around 3% globally. Butadiene is commonly produced through steam cracking, direct dehydrogenation of n-butane and n-butene, and oxidative dehydrogenation of n-butene. The first route accounts for over 95 % of global butadiene production.

In contrast thereto the production of 1,3 butadiene (1,3-BDE) - in the present specification just "butadiene" - from bio-renewable resources of 2,3-butanediol (2,3-BDO) by catalytic dehydration is in the focus as 2,3-BDO is readily obtained from biomass or its derivates, for instance fermentation of xylose and glucose.

As a consequence extensive catalyst screening started to identify suitable catalysts with promising butadiene yield. Several solid acid and base catalysts were tested, including microporous/mesoporous molecular sieves, metal phosphates, rare earth oxides, mixed oxides ($SiO_2$-$Al_2O_3$, $CeO_2$-$Al_2O_3$, $ZrO_2$-$Al_2O_3$, etc), supported heteropolyacids (HPAs), solid super-acids (sulfated-$ZrO_2$, Tungstated-$ZrO_2$), double hydroxides, alkali metals supported on metal oxides, among others.

Dehydration of 2,3-BDO leads to either 2-butanone (MEK) or isobutyraldehyde (MPA) or 1,3-BDE. The reaction is exothermic for the first two and these are therefore preferred in many reported data. Achieving high 1,3-BDE selectivity is challenging because this reaction is endothermic. Therefore, the development of novel catalysts and/or catalytic processes with improved selectivity towards butadiene is of prime importance for its commercial production. DE 2 117 444 A1 provides a method of preparing dehydration catalysts comprising at least one neutral pyrophosphate of at least one metal selected from lithium, sodium, strontium and barium with at least one acid ortho-phosphate of at least one metal selected from lithium, sodium, strontium and barium, forming an extrudate from the resulting mixture and thereafter drying and calcining the extrudate in air in order to convert the acid orthophosphate into neutral pyrophosphate and their use for dehydration of dioles. The only catalyst described in DE 2 117 444 A1 consisted primarily of a mixture of lithium sodium double pyrophosphate and sodium pyrophosphate.

KR101298672 B1 describes a manufacturing method of 1,3-butadiene from 2,3 - butanediol. The catalysts used are $Ca_2P_2O_7$ and $CaNa_2P_2O_7$.

Most of the mono-metal oxides, mixed oxides, zeolites (including dealuminated zeolites) and some typical strong solid acids (Heteropolyacid (HPA) and acid resin) are not selective to 1,3-BDE. Normally lower than 10% selectivity to 1,3-BDE is obtained. MEK is the main product regardless of the catalyst is a base or acid oxide.

## Object of the invention

It was therefore an object of the present invention to identify promising novel catalysts based on alkali earth metals for the production of butadiene by dehydration of 2,3-BDO, targeting a 1,3-BDE selectivity $\geq$ 40 % and a 2,3-BDO conversion $\geq$ 90 %.

The object was achieved by using at least one catalyst selected from the group of

a) mixed alkaline earth metal pyrophosphate wherein at least one alkaline earth metal is magnesium or strontium,

b) lithium alkaline earth metal phosphate and

c) lithium alkaline earth metal pyrophosphate.

## Summary of the invention

The present invention refers to the dehydration of 2,3-butanediol to butadiene wherein at least one catalyst selected from the group of

a) mixed alkaline earth metal pyrophosphate wherein at least one alkaline earth metal is magnesium or strontium,

b) lithium alkaline earth metal phosphate and

c) lithium alkaline earth metal pyrophosphate.

is used.

For clarification, it should be noted that the scope of the invention encompasses all of the definitions and parameters listed in general terms or in preferred ranges in the present specification, in any desired combination.

Preferred embodiments of the invention

Preferred catalysts are selected from components a) and c).

Most preferred catalysts are selected from component a).

Preferred alkaline earth metals to be used in components a) to c) are magnesium, calcium, strontium or barium, more preferred are calcium or strontium.

Preferred catalysts of component a) contain combinations of

    i) magnesium and calcium, or

    ii) magnesium and strontium, or

    iii) calcium and strontium,
    especially iii) calcium and strontium.

Preferred catalysts of component b) comprise barium as alkaline earth metal.

Preferred catalysts of component c) comprise barium or calcium as alkaline earth metal, more preferred barium.

Preferred calcium containing catalysts of component a) contain calcium in the range of from 50 to 75 mole-% with respect to the total earth alkali metal content of the catalyst.

In case that the catalyst according to component a) contains no calcium but strontium, the strontium content is preferably in the range of from 25 to 75 mole-% with respect to the total earth alkali metal content.

Especially preferred catalysts of component a) are characterized by the formulas $(Mg_xCa_{1-x})_2P_2O_7$, $(Mg_xSr_{1-x})_2P_2O_7$, $(Ca_xSr_{1-x})_2P_2O_7$ and $(Sr_xBa_{1-x})_2P_2O_7$, where x = 0.10 - 0.8.

Especially most preferred catalysts of component a) are $(Mg_{0,5}Ca_{0,5})_2P_2O_7$, $(Mg_{0,25}Ca_{0,75})_2P_2O_7$, $Na_2(Mg_{0,5}Ca_{0,5})_2P_2O_7$, $(Mg_{0,25}Sr_{0,75})_2P_2O_7$ and $(Ca_{0,75}Sr_{0,25})_2P_2O_7$. More preferred catalysts are selected from the group of $LiBaPO_4$, $Li_2BaP_2O_7$, $Li_2CaP_2O_7$, $(Mg_{0,5}Ca_{0,5})_2P_2O_7$, $(Mg_{0,25}Ca_{0,75})_2P_2O_7$, $Na_2(Mg_{0,5}Ca_{0,5})_2P_2O_7$, $(Mg_{0,25}Sr_{0,75})_2P_2O_7$ and $(Ca_{0,75}Sr_{0,25})_2P_2O_7$.

Most preferred catalysts are selected from the group $Na_2(Mg_{0,5}Ca_{0,5})_2 P_2O_7$, $(Mg_{0,25}Sr_{0,75})_2 P_2O_7$ and $(Ca_{0,75}Sr_{0,25})_2 P_2O_7$.

Especially preferred catalysts are $Na_2(Mg_{0,5}Ca_{0,5})_2 P_2O_7$ and $(Ca_{0,75}Sr_{0,25})_2 P_2O_7$.

Especially most preferred $(Ca_{0,75}Sr_{0,25})_2 P_2O_7$ is used as a catalyst for the dehydration of 2,3-butanediol to butadiene.

Process to manufacture the catalysts

Catalysts according to the present invention were prepared by solid state reaction and wet chemical methods, preferably solid state reaction and co-precipitation methods.

Catalysts of component a)

Mixed alkaline earth metal pyrophosphates, preferably catalysts of the general formulas $(Mg_xCa_{1-x})_2P_2O_7$, $(Mg_xSr_{1-x})_2P_2O_7$, $(Ca_xSr_{1-x})_2P_2O_7$ and $(Sr_xBa_{1-x})_2P_2O_7$, where x = 0.125 - 0.75, are synthesized by a co-precipitation method, preferably by mixing stoichiometric aqueous solutions of precursors followed by thermal treatment.

Coprecipitation, together with precipitation, is the most frequently applied method for the preparation of unsupported catalysts. However, both methods have the major disadvantage of forming large volumes of salt-containing solutions in the precipitation stage and in washing the precipitate. Source materials are mainly metal salts, such as sulfates, chlorides, and nitrates. Acetates, formiates, or oxalates are used in some cases. In industrial practice nitrates or sulfates are preferred. Basic precipitation agents on an industrial scale are hydroxides, carbonates, and hydroxocarbonates of sodium, potassium, or ammonium. Alkali metal nitrates or sulfates formed as precipitation byproducts must be washed out of the precipitate. Thermally decomposable anions, such as carbonates and carboxylates and cations like $NH_4^+$ are especially favored in catalyst production.

Coprecipitation of two or more metal cations is a suitable operation for the homogeneous dispersion of the corresponding oxides, phosphates or pyrophosphates, especially if the catalyst precursors have a defined crystalline structure, for example, $Cu(OH)NH_4CrO_4$ or $Ni_6Al_2(OH)_{16}CO_3$. After thermal treatment, binary oxides, phosphates or pyrophosphates such as $CuO$ - $Cr_2O_3$ and $NiO$ - $Al_2O_3$ are formed (Deutschmann, Knözinger, Kochloefl, Turek: Heterogeneous Catalysis and Solid Catalysts, 2. Development and Types of Solid Catalysts, in Ullmann's Encyclopedia of Industrial Chemistry, Wiley-VCH Verlag, 2000, p. 504).

Catalysts of component b) and c)

$LiMPO_4$ catalysts, where M = Mg, Ca, Ba, as well as lithium alkaline earth metal pyrophosphates, were prepared by solid-state reaction technique (BM). High purity of $Li_2CO_3$, $MgCO_3$, $CaCO_3$, $BaCO_3$ and $(NH_4)_2HPO_4$ are used as precursors. Stoichiometric quantities of chemical precursors are mixed by ball-milling with $ZrO_2$ media in an alcohol and calcined.

Mechanical Treatment, for example, mixing, ball milling, or kneading of catalytic active materials or their precursors with promoters, structure stabilizers, or pore-forming agents, is one of the simplest catalyst preparation methods. If the required intimate contact of catalyst components is achieved, the activity, selectivity, or thermal stability of catalysts prepared in this way will be equal to those prepared by other methods. However, recent improvements in the efficiency of various aggregates for the mechanical treatment of solids resulted in activity enhancement. An important advantage of these methods is that formation of wastewater is avoided. (Deutschmann, Knözinger, Kochloefl, Turek: Heterogeneous Catalysis and Solid Catalysts, 2. Development and Types of Solid Catalysts, in Ullmann's Encyclopedia of Industrial Chemistry, Wiley-VCH Verlag, 2000, p. 503).There are several conditions under which a solid state reaction can take place. Oven techniques use high temperatures to encourage reactions without solvents. In a melt technique, the reactants are melted together. The melted reactants interact in the liquid state and become a paste which then hardens into a solid. See also: http://en.wikipedia.org/wiki/Solid-state_reaction_route.

In a further embodiment, depending on the manufacturing process, the catalysts according to the invention further comprise at least one transition metal compound, said transition metal compounds are preferably selected from oxygen compounds of the elements titanium, in particular titanium (IV) oxide, vanadium, in particular vanadium (V) oxide, molybdenum, tungsten, manganese , in particular manganese (II) oxide, copper, in particular copper (II) oxide, nickel, in particular nickel (II) oxide, zinc, in particular zinc (II) oxide or chromium, in particular chromium (III) oxide.

In one embodiment, the catalysts according to the invention contain oxygen compounds of magnesium, zircon, aluminum or silicon or alternatively or additionally, inert materials that do not react under the reaction conditions of the dehydration process with the reactants or products. Such inert materials are preferably graphite, silicon carbide or boron nitride.

In one embodiment, the catalysts of the invention contain alkali metal oxides, calculated on $M_2O$ wherein M is an alkali metal, in particular lithium, sodium or potassium, preferably less than 0.1 wt.-% , more preferred less than 0.08 wt. - % based on 100 wt.-% catalyst.

Dehydration process

In the dehydration process liquid 2,3-BDO is constantly fed into a fixed bed reactor, containing the catalyst at 380 - 450 °C. Simultaneously, if desired, an inert gas, preferably nitrogen, is fed into the reactor as a carrier gas. The concentration of 2,3-BDO ranges from 3 % to 21.6 % of the total gas feed volume The absolute pressure inside the reactor can range from atmospheric to 3 bar. The amount of catalyst inside the reactor is related to the feed rate of 2,3-BDO, so that WHSV (weight hourly space velocity, i.e. the mass of fed 2,3-BDO per hour, devided by the catalyst mass) ranges from 0.5 $h^{-1}$ to 1.5 $h^{-1}$.

The present invention even refers to novel catalysts of the formula $LiMPO_4$, $Li_2MP_2O_7$, $M1M2P_2O_7$ or $Na_2(M1M2)P_2O_7$ wherein M, M1 and M2 independent from each other represent an alkali earth metal selected from the group magnesium, calcium, barium and strontium.

Preferred catalysts are those represented by the formulas $(Mg_xCa_{1-x})_2P_2O_7$, $(Mg_xSr_{1-x})_2P_2O_7$, $(Ca_xSr_{1-x})_2P_2O_7$ and $(Sr_xBa_{1-x})_2P_2O_7$, where x = 0.10 - 0.80.

The present invention even refers to a process for the manufacture of the novel catalysts by solid state reaction and wet chemical methods, preferably solid state reaction and co-precipitation methods.

The present invention is even directed to the use of catalysts of the formula $LiMPO_4$, $Li_2MP_2O_7$, $M1M2P_2O_7$ or $Na_2(M1 M2)P_2O_7$, wherein M, M1 and M2 independent from each other represent an alkali earth metal selected from the group magnesium, calcium, barium and strontium, for the dehydration of 1,3-butanediol to butadiene.

Examples

Catalyst preparation

Catalysts of component b)

In a typical synthesis, 10.0 g $Na_4P_2O_7$ was first dissolved into 150 ml $H_2O$ at 70 °C followed by addition of stoichiometric Mg, Ca, Sr and Ba nitrates in 100 ml $H_2O$ by pouring. The white precipitate was kept overnight at room temperature under the constant stirring. Subsequently the precipitate was collected by centrifugation followed by washing thoroughly with deionized water to remove sodium residue, and dried at 80 °C. Finally, the alkaline earth metal pyrophosphates were obtained by calcination in air at 500 °C for 4 h.

Catalysts of component c)

$LiMPO_4$ catalysts, where M = Mg, Ca, Ba, were prepared by solid-state reaction technique. High purity of $Li_2CO_3$, $MgCO_3$, $CaCO_3$, $BaCO_3$ and $(NH_4)_2HPO_4$ were used as precursors. Stoichiometric quantities of chemical precursors were mixed by ball-milling with $ZrO_2$ media in ethanol for 10 h at 200 rpm. The dried samples were calcined in flowing $N_2$ at 500 °C for 6 h with a ramp rate of 5 °C/min. The final products were taken for analyses.

Dehydratation process

2,3-BDO was fed into the reactor at a rate of 0.005 mL/min, together with 20 sccm of nitrogen as a carrier gas. 0.2 g of catalyst were used (sccm = standard cubic centimeter per minute). Thus, partial pressure of 2,3-BDO was 5.78 % and WHSV was 1.48 $h^{-1}$. WHSV (weight hourly space velocity) is the mass of fed 2,3-BDO per hour, divided by the catalyst mass.

The catalyst screening in the present invention was carried out in a fixed bed reactor system comprising of a reactant feed, a reactor and a detector. The liquid feed to the reactor was delivered via a Shimadzu LC-20AT liquid pump while gas feed was delivered using a Brooks Instrument mass flow control system. The reactor module consisted of stainless steel tubular reactors placed vertically within a tube furnace, with a diameter of 3/8" used for packing 0.2 g of catalyst. Detection of chemical species was performed using an Agilent Technologies 7890A gas chromatography system (GC). Stainless steel tubing and fittings were used for the reactor system and procured from Swagelok®.

Under normal operating conditions, 2,3-BDO was fed at a rate of 0.005 mL/min, together with 20 sccm of $N_2$ as a carrier gas and 0.2 g of catalyst. This combination gives a 2,3-BDO partial pressure composition of 5.78% and a weight hourly space velocity (WHSV) of 1.48 $h^{-1}$. Chemical species were identified via their GC retention time via the flame ionization detector (FID). These values are given in **Table 1.**

**Table 1:** GC FID retention time (in min) for reactant and product species for system 1

| Methane | Ethylene | Ethane | Propylene | Propane |
|---|---|---|---|---|
| 1.23 | 1.34 | 1.39 | 1.84 | 1.93 |
| Acetaldehyde | Iso-butylene | 1,3-BDE | Trans-2-butene | Cis-2-butene |
| 2.34 | 3.11 | 3.21 | 3.41 | 3.54 |
| Acetone | 2-propanol | MPA | MVC | 2,3-BDOne |
| 4.54 | 6.21 | 6.80 | 7.50 | 7.70 |
| MEK | Iso-butanol | 1-butanol | Acetoin | 2,3-BDO |
| 7.84 | 9.10 | 9.70 | 11.80 | 13.20 |

**Calculation of conversion**

Conversion of BDO (butanediol) is defined as follow:

$$Conversion\ (\%) = 1 - \frac{Outlet\ flowrate\ of\ BDO\ (gas)\ \left[\frac{ml}{min}\right]}{Initial\ flowrate\ of\ BDO\ (gas)\ \left[\frac{ml}{min}\right]}\ x\ 100 \quad (1)$$

According to the above definition of conversion in eq. (1), the conversion of BDO is calculated based on the FID area of initial and unreacted BDO since the reactant and product were very diluted, the change in total gas flowrate before and after the reaction was very small.

$$X_{2,3-BDO} = 1 - \frac{A}{A_i} \quad (2)$$

where $X_{2,3-BDO}$ is the BDO *conversion, A* is the unreacted BDO area and *Ai* is the initial area of BDO.

**Calculation of selectivity**

The selectivity to various products was calculated based on FID peak areas corrected for their internal response factor (IRF). IRF values used are shown in **Table 2.**

**Table 2:** IRF values used for various chemical species (Dietz 1967)

| Methane | Ethylene | Ethane | Propylene | Propane |
|---|---|---|---|---|
| 1.00 | 1.00 | 1.00 | 1.00 | 1.00 |
| **Acetaldehyde** | **Iso-butylene** | **1,3-BDE** | **Trans-2-butene** | **Cis-2-butene** |
| 1.00 | 1.00 | 1.00 | 1.00 | 1.00 |
| **Acetone** | **2-propanol** | **MPA** | **MVC** | **2,3-BDOne** |
| 1.00 | 1.00 | 1.61 | 1.00 | 1.00 |
| **MEK** | **Iso-butanol** | **1-butanol** | **Acetoin** | **2,3-BDO** |
| 1.64 | 1.47 | 1.00 | 1.00 | 1.00 |

**Mass balance**

The mass balance was calculated based on the following equation:

$$\frac{collected\ liquid + residue\ on\ catalyst + total\ gases}{mass\ of\ 2,3-BDO\ fed} \quad (3)$$

The determination of each component in the numerator is described below.

**Collected liquid**

The liquid product from the reaction was collected and weighed.

**Residue on catalyst**

The spent catalyst was burned at 700 °C for 2 hours. The weight difference before and after burning was taken as the residue on catalyst.

**Mass of BDO fed**

Mass of BDO fed to the reactor was obtained by taking the difference between the weight of BDO before and after the reaction.

**Total gases**

**Table 3:** FID area and known gas products

|  | Methane | Ethylene | Ethane | Propylene | Propane | 1,3-BDE | trans-2-butene | cis-2-butene |
|---|---|---|---|---|---|---|---|---|
| **FID Area** | 1137.5 | 1065.1 | 1040.2 | 1233.90 | 1247.60 | 2834.20 | 3275.40 | 1585.7 |
| **Concentration (%)** | 2.5 | 2.5 | 5 | 2 | 2 | 4 | 4 | 2 |

Using the values in **Table 3,** the concentration FID factor of each gas produced can be determined using the following relationship. For example 1,3-BDE:

$$Concentration\ of\ BDE\ (\%) = \frac{FID\ peak\ area\ of\ BDE\ produced}{2834,2} \times 4 \qquad (4)$$

Mass of each gas produced can be determined using the following relationship:

$$\frac{Gas\ concentration \times total\ gas\ flowrate \left[\frac{ml}{min}\right] \times run\ time\ [min] \times \frac{1}{1000} \left[\frac{l}{ml}\right] \times molecular\ weigth\ \frac{g}{mol}}{22,4\ l/mol}$$

$$(5)$$

The total mass of gases can then be determined by summing up all the mass of gases produced.

**Results**

Catalysts according to the present invention were prepared by solid state reaction and wet chemical methods. Ten potential catalysts with selectivity of 1,3-BDE more than 40% were identified and listed in **Table 4.** The best catalyst is $(Ca_{0.75}Sr_{0.25})_2P_2O_7$. Its selectivity over 1,3-BDE is stable at 59% with full conversion of 2,3-BDO. $(Na_2(Mg_{0.5}Ca_{0.5})P_2O_7$ also has stable performance at 450 °C with 51% selectivity to 1,3-BDE and 100% conversion of 2,3-BDO.

**Table 4**

| | Catalyst | Synthesis Method | 1,3-BDE Selectivity (%Average) | BDO Conversion (%) | T (°C) | BDO-concentration in the feed (%) | WHSV (h$^{-1}$) |
|---|---|---|---|---|---|---|---|
| 1 | $LiBaPO_4$ | BM | 40 | 100 | 400 | 5.85 | 1.5 |
| 2 | $Li_2BaP_2O_7$ | BM | 41 | 100 | 400 | 5.85 | 1.5 |
| 3 | $Li_2CaP_2O_7$ | Co-ppt | 40 | 96 | 400 | 5.85 | 1.5 |
| 4 | $(Mg_{0.5}Ca_{0.5})_2P_2O_7$ | Co-ppt | 45 | 100 | 380-400 | 5.85 | 1.5 |
| 5 | $(Mg_{0.25}Ca_{0.75})_2P_2O_7$ | Co-ppt | 45 | 90 | 450 | 5.85 | 1.5 |
| 6 | $Na_2(Mg_{0.5}Ca_{0.5})P_2O_7$ | Co-ppt | 51 | 100 | 450 | 5.85 | 1.5 |
| 7 | $(Mg_{0.25}Sr_{0.75})_2P_2O_7$ | Co-ppt | 46 | 100 | 380-400 | 5.85 | 1.5 |
| 8 | $(Ca_{0.75}Sr_{0.25})_2P_2O_7$ | Co-ppt | 59 | 100 | 430 | 5.85 | 1.5 |

**Claims**

1. A process for the dehydration of 2,3-butanediole to butadiene **characterized in that** at least one catalyst selected from the group of

   a) mixed alkaline earth metal pyrophosphate wherein at least one alkaline earth metal is magnesium or strontium,
   b) lithium alkaline earth metal phosphate and
   c) lithium alkaline earth metal pyrophosphate is used.

2. A process according to Claim 1, **characterized in that** the catalyst is selected from the component a) and c), preferably a).

3. A process according to Claim 1 or 2, **characterized in that** the alkaline earth metals to be used in components a) to c) are magnesium, calcium, strontium or barium, more preferred are calcium or strontium.

4. A process according to any of Claims 1 to 3, **characterized in that** catalysts of component a) contain combinations of

   i) magnesium and calcium, or
   ii) magnesium and strontium, or
   iii) calcium and strontium,
   especially iii) calcium and strontium.

5. A process according to Claim 1, **characterized in that** catalysts of component b) comprise barium as alkaline earth metal.

6. A process according to any of Claims 1 to 3, **characterized in that** catalysts of component c) comprise barium or calcium as alkaline earth metal, more preferred barium.

7. A process according to any of Claims 1 to 4, **characterized in that** calcium containing catalysts of component a) contain calcium in the range of from 50 to 75 % by weight with respect to the total earth alkali metal content of the catalyst.

8. A process according to any of Claims 1 to 4, **characterized in that** the catalyst according to component a) contains no calcium but strontium, the strontium content is in the range of from 25 to 75 mole-% with respect to the total earth alkali metal content.

9. A process according to any of Claims 1 to 4, **characterized in that** catalysts of component a) are **characterized by** the formulas $(Mg_xCa_{1-x})_2P_2O_7$, $(Mg_xSr_{1-x})_2P_2O_7$, $(Ca_xSr_{1-x})_2P_2O_7$ and $(Sr_xBa_{1-x})_2P_2O_7$, where x = 0.125 - 0.75.

10. A process according to Claim 9, **characterized in that** the catalysts of component a) are $(Mg_{0,5}Ca_{0,5})_2P_2O_7$, $(Mg_{0,25}Ca_{0,75})_2P_2O_7$, $Na_2(Mg_{0,5}Ca_{0,5})_2P_2O_7$, $(Mg_{0,25}Sr_{0,75})_2P_2O_7$ and $(Ca_{0,75}Sr_{0,25})_2P_2O_7$.

11. A process according to Claim 1, **characterized in that** the catalysts are selected from the group of $LiBaPO_4$, $Li_2BaP_2O_7$, $Li_2CaP_2O_7$, $(Mg_{0,5}Ca_{0,5})_2P_2O_7$, $(Mg_{0,25}Ca_{0,75})_2P_2O_7$, $Na_2(Mg_{0,5}Ca_{0,5})_2P_2O_7$, $(Mg_{0,25}Sr_{0,75})_2P_2O_7$ and $(Ca_{0,75}Sr_{0,25})_2P_2O_7$.

12. A process according to Claim 1, **characterized in that** the catalysts are selected from the group $Na_2(Mg_{0,5}Ca_{0,5})_2P_2O_7$, $(Mg_{0,25}Sr_{0,75})_2 P_2O_7$ and $(Ca_{0,75}Sr_{0,25})_2P_2O_7$, preferably $Na_2(Mg_{0,5}Ca_{0,5})_2P_2O_7$ and $(Ca_{0,75}Sr_{0,25})_2P_2O_7$, most preferred is $(Ca_{0,75}Sr_{0,25})_2P_2O_7$.

13. Catalysts of the formula $LiMPO_4$, $Li_2MP_2O_7$, $M1M2P_2O_7$ or $Na_2(M1M2)P_2O_7$ wherein M, M1 and M2 independent from each other represent an alkali earth metal selected from the group magnesium, calcium, barium and strontium.

14. Catalysts according to Claim 13, **characterized in that** those are represented by the formulas $(Mg_xCa_{1-x})_2P_2O7$, $(Mg_xSr_{1-x})_2P_2O_7$, $(Ca_xSr_{1-x})_2P_2O_7$ and $(Sr_xBa_{1-x})_2P_2O_7$, where x = 0.1 - 0.8.

15. Use of catalysts according to Claims 13 or 14 for the dehydration of 1,3-butanediol to butadiene.

**EUROPEAN SEARCH REPORT**

Application Number

EP 14 18 0473

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GB 595 035 A (CELANESE CORP) 25 November 1947 (1947-11-25) * the whole document * | 1-12,15 | INV. C07C1/24 B01J27/18 |
| A,D | DE 21 17 444 A1 (COMPAGNIE FRANCAISE DE RAFFINAGE, S.A.) 28 October 1971 (1971-10-28) * the whole document * | 1-12,15 | |
| X | IVANOVA D ET AL: "Flux growth conditions of Li2BaP2O7 single crystals", MATERIALS LETTERS, NORTH HOLLAND PUBLISHING COMPANY. AMSTERDAM, NL, vol. 56, no. 5, 1 November 2002 (2002-11-01), pages 787-792, XP004388935, ISSN: 0167-577X, DOI: 10.1016/S0167-577X(02)00614-6 * LiBa(PO4), p. 790, left col., 2nd full paragraph * | 13 | |
| X | US 3 484 383 A (HOFFMAN MARY V) 16 December 1969 (1969-12-16) * column 2 - column 4; claim 1 * | 13,14 | TECHNICAL FIELDS SEARCHED (IPC) C07C B01J |
| X | GB 1 255 025 A (GTE SYLVANIA INC [US]) 24 November 1971 (1971-11-24) * page 1, line 47 - line 55 * | 13,14 | |
| X | MCCAULEY R A ET AL: "Phase Equilibria and Eu<2+>-, Tb<3+>-, and Mn<2+>-Activated Luminescent Phases in the CaO-MgO-P2O5 System", JOURNAL OF THE ELECTROCHEMICAL SOCIETY,, vol. 118, no. 5, 1 May 1971 (1971-05-01), pages 755-759, XP001262734, * paragraph titled "pyrophosphate join"; page 757 * | 13,14 | |

The present search report has been drawn up for all claims

| Place of search | Date of completion of the search | Examiner |
|---|---|---|
| The Hague | 9 February 2015 | Fritz, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 14 18 0473

This annex lists the patent family members relating to the patent documents cited in the above-mentioned European search report.
The members are as contained in the European Patent Office EDP file on
The European Patent Office is in no way liable for these particulars which are merely given for the purpose of information.

09-02-2015

| Patent document cited in search report | | Publication date | Patent family member(s) | | Publication date |
|---|---|---|---|---|---|
| GB 595035 | A | 25-11-1947 | NONE | | |
| DE 2117444 | A1 | 28-10-1971 | AT | 313861 B | 11-03-1974 |
| | | | AT | 318548 B | 25-10-1974 |
| | | | BE | 765090 A1 | 30-09-1971 |
| | | | CA | 975383 A1 | 30-09-1975 |
| | | | CS | 161903 B2 | 10-06-1975 |
| | | | DE | 2117444 A1 | 28-10-1971 |
| | | | FR | 2087011 A5 | 31-12-1971 |
| | | | GB | 1355704 A | 05-06-1974 |
| | | | GB | 1355705 A | 05-06-1974 |
| | | | JP | S549148 B1 | 21-04-1979 |
| | | | LU | 62940 A1 | 26-08-1971 |
| | | | NL | 7105151 A | 19-10-1971 |
| | | | RO | 57596 A | 15-04-1975 |
| | | | SE | 372512 B | 23-12-1974 |
| | | | SU | 555832 A3 | 25-04-1977 |
| | | | SU | 562191 A3 | 15-06-1977 |
| | | | US | 3781222 A | 25-12-1973 |
| | | | ZA | 7102362 A | 26-01-1972 |
| US 3484383 | A | 16-12-1969 | GB | 1177196 A | 07-01-1970 |
| | | | US | 3484383 A | 16-12-1969 |
| GB 1255025 | A | 24-11-1971 | DE | 1813967 A1 | 10-07-1969 |
| | | | FR | 1595004 A | 08-06-1970 |
| | | | GB | 1255025 A | 24-11-1971 |
| | | | US | 3516940 A | 23-06-1970 |

EPO FORM P0459

**EP 2 952 498 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- DE 2117444 A1

- KR 101298672 B1

**Non-patent literature cited in the description**

- Heterogeneous Catalysis and Solid Catalysts, 2. Development and Types of Solid Catalysts. **DEUTSCHMANN ; KNÖZINGER ; KOCHLOEFL ; TUREK.** Ullmann's Encyclopedia of Industrial Chemistry. Wiley-VCH Verlag, 2000, 504

- Heterogeneous Catalysis and Solid Catalysts, 2. Development and Types of Solid Catalysts. **DEUTSCHMANN ; KNÖZINGER ; KOCHLOEFL ; TUREK.** Ullmann's Encyclopedia of Industrial Chemistry. Wiley-VCH Verlag, 2000, 503